# EUROPEAN PATENT SPECIFICATION

(11) **EP 0 653 419 B1**
(45) Date of publication and mention of the grant of the patent: **17.09.1997**
(21) Application number: 94307946.7
(22) Date of filing: 28.10.1994
(51) Int. Cl.: C07C 243/28

(54) **Preparation of pivaloyl hydrazide**
Herstellung von Pivaloylhydrazid
Préparation d'hydrazide de l'acide pivaloique

(30) Priority: 11.11.1993 EP 93309014
(43) Date of publication of application: 17.05.1995
(73) Proprietor: SHELL INTERNATIONALE RESEARCH MAATSCHAPPIJ B.V., 2596 HR Den Haag (NL)
(72) Inventor: Verbrugge, Pieter Adriaan, NL-1503 WC Zaandam (NL); De Waal, Jannetje, NL-1131 HM Volendam (NL)
(74) Representative: Allam, Peter Clerk

(56) References cited:
- DE-A- 2 364 059

## Description

The present invention relates to the preparation of pivaloyl hydrazide by reaction between pivalic acid and hydrazine.

GB-A-1396615 discloses the condensation of a carboxylic acid with ammonia, hydrazine or substituted hydrazine or a primary or secondary amine with a catalyst M(OR)ₙX₍₄₋ₙ₎ where M is titanium or zirconium, R is alkyl or aryl, X is halogen and n is 1-4. There is, however, in that document no reference to nor example of pivalic acid as the carboxylic acid. There are only examples of less hindered acids, namely nicotinic, iso-nicotinic, benzoic and acetic acids. Moreover, in all of the examples in which hydrazine is the other reactant, water is azeotroped out before addition of the catalyst, presumably with the intention of avoiding hydrolysis of the alkoxide group in the catalyst.

It has, however, now been found that if the process exemplified in GB-A-1396615 is used for reaction of pivalic acid with hydrazine, namely in which water is removed before addition of the catalyst, lower yields of pivaloyl hydrazide are obtained than if the water is left in the mixture during such addition.

The present invention therefore provides a process for the preparation of pivaloyl hydrazide which comprises reacting pivalic acid with hydrazine in the presence of a catalytic amount of amorphous titanium dioxide in an inert solvent, in the presence of water.

The amorphous titanium dioxide is preferably prepared by addition to the pivalic acid/hydrazine mixture of a titanium compound which can form amorphous titanium dioxide upon such addition. Such titanium compound is preferably a titanium alkoxide or phenoxide, more preferably titanium tetra-isopropoxide or titanium tetra-phenoxide. Particularly good yields are obtained using titanium tetra-isopropoxide, there being no examples of such compound in GB-A-1396615.

The molar percent of said titanium compound to the pivalic acid is preferably in the range of from 0.5 to 2.

The solvent may be any suitable inert solvent, including in particular 2-methyl-1-butanol, n-butanol and s-butanol, in some circumstances together with toluene. 2-methyl-1-butanol is particularly preferred.

The molar ratio of the hydrazine compound to the pivalic acid is preferably in the range of from 1.05 to 1.2.

During the process the reaction mixture is preferably heated to its reflux temperature.

Comparative tests have shown in fact that if crystalline titanium dioxide is used as catalyst instead of amorphous titanium dioxide, substantially inferior results are obtained.

In order to demonstrate the efficacy of the process of the present invention, a number of Examples are set out below. Examples 1 to 6 describe processes carried out according to the present invention, whilst Example 7 describes the use of a comparative process.

### EXAMPLES 1 TO 5

Pivalic acid was dissolved in the solvent (solvent/pivalic acid - 1.2 w/w) and hydrazine hydrate (hydrazine hydrate/pivalic acid - 1.2 mol/mol) was added. Whilst being mechanically stirred, the reaction mixture was heated and the titanium compound added. The temperature was then further raised to reflux via a Dean Stark water separator. During this reaction, the boiling point of the reaction increased. When water separation stopped and the boiling point temperature remained constant, no further conversion could be expected. After cooling, the catalyst was filtered and the filtrate boiled down.

The results are set out in Table 1. The yield figures given in the table are of the flashed product. The figures in brackets under each titanium compound indicates the molar percent with respect to the pivalic acid.

**TABLE 1**

| Ex. No. | Solvent ** | Titanium Compound *** | Reaction time (hours) | % Conversion of pivalic acid | Product yield % * |
|---|---|---|---|---|---|
| 1 | 2-Me-1-BuOH | Ti(O-iPr)₄ (1) | 2 | >99 | 93 |
| 2 | 2-Me-1-BuOH | Ti(O-iPr)₄ (0.5) | 3 | >99 | 95 |
| 3 | sec-BuOH + | Ti(O-iPr)₄ (0.5) | 6 | >99 | 94 |
| 4 | sec-BuOH | Ti(O-iPr)₄ (0.5) | 7.5 ++ | >99 | 96 |
| 5 | 2-Me-1-BuOH | Ti(O-Ph)₄ (1) | 3 | >99 | 92 |

| | | | | | |
|---|---|---|---|---|---|
| * pivaloyl hydrazide | | | | | |
| ** 2-Me-1-BuOH = 2-methyl-1-butanol; sec-BuOH = sec. butanol | | | | | |
| *** Ti(OPh)₄ = titanium tetra-phenoxide; Ti(O-iPr)₄ = titanium tetra-isopropoxide | | | | | |
| + plus sufficient cyclohexane to induce water separation | | | | | |
| ++ refluxed 4 hours without Dean Stark separator, then 3.5 hours under replenishment with fresh sec-BuOH | | | | | |

### Example 6

(a) Hydrazine hydrate (800 mmol), titanium tetra-isopropoxide (33.5 mmol), n-butanol (90 ml) and toluene (50 ml) were azeotroped dry (i.e. no pivalic acid was present) and after 2 additional hours boiling cooled down and the amorphous titanium dioxide precipitate isolated by filtration and dried under vacuum.
(b) To pivalic acid (200 mmol) and hydrazine hydrate (215 mmol) in n-butanol (30 ml) and toluene (15 ml) was added 3.75 mmol of the isolated amorphous titanium dioxide and the mixture azeotroped dry. 100% conversion to pivaloyl hydrazide was reached in 2 hours. After 3 hours, the mixture was cooled down and the precipitate filtered over 'Hyflo' filter aid. Flashing of the filtrate left 100% pure pivaloyl hydrazide in 97% yield.

### EXAMPLE 7

The process conditions of Example 1 of GB-A-1396615 were applied to the reaction between pivalic acid and hydrazine hydrate as follows.

Pivalic acid (480 mmol) and hydrazine hydrate (516 mmol) in n-butanol (65 ml) and toluene (35 ml) were azeotroped dry via a Dean Stark separator (10 ml lower layer) before titanium tetra-isopropoxide (9 mmol) (i.e. 1.9 molar percent with respect to pivalic acid) was added. Reflux was continued until a constant boiling point of 114°C (19 ml lower phase out) was achieved. When, after 6 hours, conversion did not increase beyond 90 molar percent, the mixture was worked up to leave a product containing 91% molar percent pivaloyl hydrazide, 2.5 molar percent dipivaloyl hydrazide and 5 molar percent pivalic acid, the remainder being retained solvent.

It can be seen that the yield of the product pivaloyl hydrazide obtained by the process of the present invention is clearly greater than that obtained using the prior art process conditions.

In order to show the lower yield using crystalline titanium dioxide as catalyst, we set out in Table 2 the results of experiments carried out as described above for Examples 1 to 5.

**TABLE 2**

| Ex. No. | Solvent | Catalyst | Reaction time (hours) | % Conversion of pivalic acid | Contained yield % |
|---|---|---|---|---|---|
| 8 | 2-Me-1-BuOH | TiO₂ (1) | 6 | 88 | 82 |
| 9 | 2-ME-1-BuOH | TiO₂ (1) | 8 | 90 | 72 |
| 10 | 2-Me-1-BuOH | TiO₂ (25) | 6.5 | 80 | 68 |

It can be seen quite clearly that the conversion of pivalic acid achieved using crystalline titanium dioxide is distinctly lower than that achieved using the process of the present invention.

## Claims

1. A process for the preparation of pivaloyl hydrazide which comprises reacting pivalic acid with hydrazine in the presence of a catalytic amount of amorphous titanium dioxide in an inert solvent, in the presence of water.

2. A process according to claim 1 in which the amorphous titanium dioxide is prepared by addition to the pivalic acid/hydrazine mixture of a titanium compound which can form amorphous titanium dioxide upon such addition.

3. A process according to claim 2 in which said titanium compound is a titanium alkoxide or phenoxide.

4. A process according to claim 3, in which said titanium compound is titanium tetra-isopropoxide or titanium tetra-phenoxide.

5. A process according to claim 4, in which said titanium compound is titanium tetra-isopropoxide.

6. A process according to any one of claims 2 to 5, in which the molar percent of said titanium compound to the pivalic acid is in the range of from 0.5 to 2.

7. A process according to any one of claims 1 to 6 in which the solvent is selected from 2-methyl-1-butanol, n-butanol and s-butanol, optionally together with toluene.

8. A process according to claim 7, in which the solvent is 2-methyl-1-butanol.

9. A process according to any one of claims 1 to 8, in which the molar ratio of the hydrazine compound to the pivalic acid is in the range of from 1.05 to 1.2.

10. A process according to any one of claims 1 to 9, in which the reaction mixture is heated to its reflux temperature.

## Patentansprüche

1. Verfahren zur Herstellung von Pivaloylhydrazid, umfassend das Umsetzen von Pivalinsäure mit Hydrazin in Anwesenheit einer katalytischen Menge an amorphem Titandioxid in einem inerten Lösungsmittel in Anwesenheit von Wasser.

2. Verfahren nach Anspruch 1, in dem das amorphe Titandioxid hergestellt wird durch Zugabe einer Titanverbindung zu der Pivalinsäure/Hydrazin-Mischung, welche bei einer derartigen Zugabe amorphes Titandioxid bilden kann.

3. Verfahren nach Anspruch 2, in dem die Titanverbindung ein Titanalkoholat oder -phenolat ist.

4. Verfahren nach Anspruch 3, in dem die Titanverbindung Titantetraisopropanolat oder Titantetraphenolat ist.

5. Verfahren nach Anspruch 4, in dem die Titanverbindung Titantetraisopropanolat ist.

6. Verfahren nach irgendeinem der Ansprüche 2 bis 5, in dem der Molprozentsatz der Titanverbindung zu der Pivalinsäure im Bereich von 0,5 bis 2 liegt.

7. Verfahren nach irgendeinem der Ansprüche 1 bis 6, in dem das Lösungsmittel aus 2-Methyl-1-butanol, n-Butanol und s-Butanol, gegebenenfalls zusammen mit Toluol, ausgewählt ist.

8. Verfahren nach Anspruch 7, in dem das Lösungsmittel 2-Methyl-1-butanol ist.

9. Verfahren nach irgendeinem der Ansprüche 1 bis 8, in dem das Molverhältnis der Hydrazinverbindung zu der Pivalinsäure im Bereich von 1,05 bis 1,2 liegt.

10. Verfahren nach irgendeinem der Ansprüche 1 bis 9, in dem die Reaktionsmischung auf ihre Rückflußtemperatur erwärmt wird.

## Revendications

1. Procédé pour la préparation de pivalohydrazide, qui comprend la réaction de l'acide pivalique avec l'hydrazine en présence d'une quantité catalytique de dioxyde de titane amorphe, dans un solvant inerte en présence d'eau.

2. Procédé selon la revendication 1, où le dioxyde de titane amorphe est préparé par addition, au mélange d'acide pivalique/hydrazine, d'un composé du titane qui peut former du dioxyde de titane amorphe lors d'une telle addition.

3. Procédé selon la revendication 2, où ledit composé du titane est un alcoolate ou un phénolate de titane.

4. Procédé selon la revendication 3, où ledit composé du titane est le tétra-isopropylate de titane ou le tétra-phénolate de titane.

5. Procédé selon la revendication 4, où ledit composé du titane est le tétra-isopropylate de titane.

6. Procédé selon l'une quelconque des revendications 2 à 5, où le pourcentage molaire dudit composé du titane à l'acide pivalique est dans la gamme de 0,5 à 2.

7. Procédé selon l'une quelconque des revendications 1 à 6, où le solvant est choisi parmi le 2-méthyl-1-butanol, le n-butanol et le sec-butanol, éventuellement avec du toluène.

8. Procédé selon la revendication 7, où le solvant est le 2-méthyl-1-butanol.

9. Procédé selon l'une quelconque des revendications 1 à 8, où le rapport molaire de l'hydrazine à l'acide pivalique est dans la gamme de 1,05 à 1,2.

10. Procédé selon l'une quelconque des revendications 1 à 9, où le mélange réactionnel est chauffé à sa température de reflux.
